# EUROPEAN PATENT SPECIFICATION

(11) **EP 2 506 827 B1**
(45) Date of publication and mention of the grant of the patent: **31.07.2013**
(21) Application number: 10787382.0
(22) Date of filing: 30.11.2010
(51) Int. Cl.: A61K 8/86, A61Q 19/00, A61K 8/81

(54) **Lubricious Aqueous Composition**
Wässrige Schmiermittelzusammensetzung
Composition aqueuse lubrifiante

(30) Priority: 30.11.2009 EP 09177537
(43) Date of publication of application: 10.10.2012
(73) Proprietor: Inoflex AB, 241 96 Stockamöllan (SE)
(72) Inventor: STERN, Leif Einar, S-241 96 Stockamöllan (SE)
(74) Representative: Ström & Gulliksson AB
(86) International application number: PCT/EP2010/068504
(87) International publication number: WO 2011/064383

(56) References cited:
- EP-A2- 1 055 425
- WO-A1-2005/030163
- WO-A1-2007/004200
- DE-A1-102004 029 328
- DE-A1-102005 014 423
- US-A- 4 790 981
- US-A1- 2005 220 828
- US-A1- 2007 249 514
- Lubrizol: "Carbopol Ultrez 21 Polymer Product Specifications", Lubrizol , 18 September 2003 (2003-09-18), XP002592599, Retrieved from the Internet: URL:http://www.lubrizol.com/WorkArea/Downl oadAsset.aspx?id=32516 [retrieved on 2010-07-15]
- Lubrizol: "Carbopol Ultrez 21 Polymer Technical Data Sheet", Lubrizol , 6 November 2002 (2002-11-06), XP002592600, Retrieved from the Internet: URL:http://www.lubrizol.com/WorkArea/Downl oadAsset.aspx?id=31982 [retrieved on 2010-07-15]
- Gottschalck & McEwen: "Acrylates/C10-30 Alkyl Acrylate CrosspolymerAcrylates/C10-30 Alkyl Acrylate Crosspolymer" In: Gottschalck & McEwen: "International Cosmetic Ingredient Dictionary and Handbook 11 Ed. 2006", 31 December 2006 (2006-12-31), The Cosmetic, Toiletry and Fragrance Association, USA, XP002592601, ISBN: 1-882621-34-4 the whole document

## Description

### Field of the Invention

This invention pertains in general to the field of compositions to be used as sex lubricants. More particularly the invention relates to a lubricious aqueous composition comprising at least one thickener and at least one compound forming a shear-thickening fluid when mixed with water.

### Background

Sex lubricants, sometimes denoted personal lubricants, are used to complement the secretions in the vagina and by the penis during sexual intercourse or during masturbation. They may be water, silicon or oil based. Water based sex lubricants in the art do typically have a water content of 15 to 85 wt%, i.e. a significant content (15 wt% or more) of components in addition to water, which will remain in the vagina after sexual intercourse and which may affect the natural flora of micro organism in the vagina.

The water in water-based sex lubricants of the art will absorb into the skin and evaporate. Eventually the sex lubricant will thus dry out, leaving a residue derived from other ingredients in the lubricant. Particularly sugar (or glycerin) and other chemicals and preservatives create a sticky residue and associated sensation, often associated with an unpleasant taste and smell.

As indicated, examples of components typically present in water based sex lubricants are glycerol, propylene glycol and cellulose derivatives, all being degradable by microorganisms and hence promoting their growth. As a consequence, sex lubricants typically comprise preservatives. However, preservatives may irritate the genitals. Lubricants containing glycerin may also promote or exacerbate vaginal yeast infections in susceptible persons.

Further, oils or lotions are commonly used as lubricants to reduce the friction between the body, such as the back, and the hands of a masseur during massage.

Massage oil does typically comprise a mixture of lipophilic components, including plant oils, such as coconut oil. Furthermore, massage oils may comprise fragrant agents as well as components having moistening effect on the skin. Massage oils may also comprise agents having a local pharmaceutical effect, such as camphor, having a weak local anesthetic and giving a cooling feeling on the skin.

Similarly, massage lotions also comprise lipophilic components.

Although commonly used massage oils and lotions are lubricious and may have a moistening effect on the skin, they suffer from several drawbacks.

Lipophilic massage oils and lotions of the art tend to stain clothes worn and/or sheets and towels used during massage, effectively leading to material damages and/or at least to an increased need of washing. Furthermore, although parts of the lubricant used during massage are absorbed by the skin, an oily residue will be left on the skin after the massage, leading to further unwanted effects. For example, this residue typically has to be washed off using soap to get rid of the oily feeling and to prevent staining of clothes. This is a clearly cumbersome process to go through after having had a nice relaxing massage. Furthermore, frequent use of such oils and lotions may induce or worsen acne. US 2007/0249541 relates to a detersive composition comprising clay. WO 07/004200 relates to a hair gel.

DE 10 2005 014 423 relates to detersive compositions comprising tensides. Similarly, WO 05/030163 relates to cosmetic and dermatological detergent preparations comprising surfactants and EP 1 055 425 relates to clear, aqueous, foamable skin cleansers comprising fatty acids amide of an amino acid, serving as cleanser.

US 2005/0220828 relates to a method of treating keratinous tissue.

DE 10 2004 029 328 relates to a transparent water phase having floating droplets of a cosmetic emulsion.

Thus there is need for a lubricious composition not suffering from the above mentioned drawbacks and being suitable as a sex lubricant.

### Summary

Accordingly, embodiments of the present invention preferably seeks to mitigate, alleviate, eliminate and/or circumvent one or more of the above-identified deficiencies in the art and disadvantages singly or in any combination and solves at least one of the above mentioned problems by providing a lubricious aqueous composition comprising at least one thickener and at least one compound forming a shear-thickening fluid when mixed with water, which solution comprises at least 95 wt% water. Wherein said thickener is a water insoluble hydrophilic compound which swells in the presence of water to form a gel, and said compound forming a shear-thickening fluid when mixed with water is a non-ionic, high molecular weight, water-soluble poly(ethylene oxide) polymer having a molecular weight of at least 100,000 Da.

Another aspect on the invention relates to use of such a lubricious aqueous composition as a sex lubricant. In such use, the composition may also serve the purpose of obstructing sperm motility. Further, such a lubricious aqueous composition may be used to obstruct sperm motility

Another aspect on the invention relates to use of such a lubricious aqueous composition for the manufacture of a sperm motility obstructing composition.

Further advantageous features of the invention are defined in the dependent claims and with regard to embodiments disclosed herein.

### Detailed description

The following description focuses on an embodiment of the present invention for use as a sex lubricant, eg. as a lubricant for sexual activities, including sexual intercourse and masturbation. In such use the sex lubricant may be applied to the vagina of a mammal, such as a human being, to the penis of a mammal, such as a human being, and/or to sex toys. However, it will be appreciated that the invention is not limited to this application but may be applied to other uses. As an example, it may be used as a pharmaceutical lubricant or during massage.

In a Newtonian fluid, the relation between shear stress and shear is linear. On the contrast, if the relation between shear stress and shear rate is nonlinear, then said fluid is denoted a "non-Newtonian" fluid. There are at least four sub-classes of non-Newtonian fluids, two of those being shear-thickening fluids, also known as dilatant fluids, and shear-thinning fluids, also known as pseudoplastic fluids.

As apparent from their names, the viscosity of shear-thinning fluids decreases with increasing shear stress. On the contrary, the viscosity of shear-thickening fluids increases with increasing shear stress.

Common examples of shear-thinning fluids are ketchup and modern paints. Corn starch in water is a commonly mentioned example of a shear thickening fluid.

An aqueous composition with high water content, such as a composition comprising at least 95 wt% water, comprising at least one thickener and at least one compound forming a shear-thickening fluid when mixed with water was shown to be suitable to be used as sex lubricant, as will be further elaborated below. Accordingly, one embodiment relates to such a lubricious aqueous composition.

Both the thickener and the compound forming a shear-thickening fluid when mixed with water should preferably be pharmaceutically acceptable. Pharmaceutically acceptable is intended to mean that the compound, at the dosage and concentrations employed, does not cause any unwanted effects. Preferably, pharmaceutically acceptable compounds are compounds approved for use as pharmaceutical excipients, such as excipients approved for use pharmaceuticals for topical administration to the skin.

As mentioned above, the water content of the lubricious aqueous composition may be at least 95 wt%, such at least 98, 99, or 99.5 wt%. The high water content of the lubricious composition implies that any residue easily is wiped off, such as with a towel, after sexual intercourse, masturbation or massage. Furthermore, it may not even be necessary to wipe off minor residues as the water anyhow will evaporate, leaving a minor amount of polymer residue. This residue was found to provide the skin with a dry velvety coating. When the lubricious aqueous composition is used as a sex lubricant, the high water content is a significant advantage, as a very limited amount of exogenous substances are administrated to the vagina.

Thickeners are typically compounds increasing the viscosity of liquids to which they are added, and thereby thickening such solutions.

The presence of a thickener will provide the lubricious aqueous composition with an increased viscosity. Thereby it may be distributed to the genitals or onto a person to be massaged effectively. Further, the presence of a thickener will imply that the composition will remain in the area of administration. As an example it will not run off the back of the person to be massaged. Without the presence of a thickener the composition will not be viscous enough to be suitable for use as a sex lubricant, at least not for controlled and unstressed use as a sex lubricant.

Preferably, the thickener may be a compound forming a shear-thinning fluid when mixed with water. By having a thickener forming a shear-thinning fluid when mixed with water, rather than any thickening agent, the lubricious aqueous composition will more easily spread to a thin layer when applied for example to a penis or to a dildo.

A composition comprising a thickening agent not forming a shear-thinning fluid when mixed with water may, according to one embodiment, be too gel-like, or viscous, to be efficiently spread.

Furthermore, the thickener may preferably be either water-soluble or a compound which swells in the presence of water to form a gel. The dissolution or swelling may require the addition of a base or an acid in order to adjust the pH of the solution or dispersion to be formed. Most preferably, the thickener is a water insoluble hydrophilic compound, which swells in the presence of water to form a gel.

As stated above it is preferred if the thickener is pharmaceutically acceptable. In relation to this, it is preferred that the thickener has such a high molecular weight that it can not penetrate human skin. Potential side effects, local as well as systemic, are minimized if the thickener never enters the body.

One example of a preferred group of thickeners to be used in a lubricious aqueous composition, as disclosed herein, is polymers of crosslinked polyacrylate, such as high weight polymers of crosslinked polyacrylate.

Crosslinked polyacrylates are in principle non-degradable by bacteria. Thus, they will not promote growth of bacteria in the vagina or disturb the natural bacterial flora. According to an embodiment, non-degradable by bacteria, i.e. non-biodegradable, is intended to mean, the bacteria are not able to degrade the compound or polymer. Thus, the compound or polymer does not serve as a nutrient.

In contrast, sex lubricants of the art, such as the sex lubricant "Klick" manufactured by RFSU (the Swedish national federation for sexual guidance), typically comprises cellulose derivates, which may act as nutrients for bacteria, acting as thickener. Furthermore, "Klick", in addition to hydroxyethyl cellulose, being a cellulose derivate, comprises glycerin and propylene glycol also serving as nutrients for bacteria. As a consequence, sex lubricants comprising components serving as nutrients for microorganisms typically also comprises preservatives. As an example, "Klick" comprises methylparaben and propylparaben. Evidently, the presence of preservatives may affect the natural flora of the vagina negatively.

By using low amounts of non-biodegradable thickeners, such as polymers of crosslinked polyacrylate, the need to add preservatives may be dispensed with.

An example of high weight polymers of crosslinked polyacrylate, are Carpopol® polymers. One especially preferred Carbopol® polymer is Carbopol® Ultrez 21 (INCI name: Acrylates/C 10-30 alkyl acrylate crosspolymer), which may obtained from Lubrizol Advanced Materials Europe BVBA in Belgium.

Crosslinked polyacrylates is an example of a group of thickeners requiring the addition of a base, such as sodium hydroxide, to swell in water. It seems as if the protonated polymer does not readily swells in water, whereas the de-protonated crosslinked polyacrylate does.

According to one embodiment, high weight polymers of crosslinked polyacrylate are intended to mean polymers, wherein the individual crosslinked polyacrylate polymer chains have an average molecular weight of at least 100,000, such as at least 250,000, as measured by gel permeation chromatography using linear polyacrylic acid as reference, if the individual polyacrylate polymer chains have been polymerized under the same conditions as the crosslinked polymer, and using the same recipe as the crosslinked grades, but without any crosslinking monomer.

The amount of the thickener in the lubricious aqueous composition, as disclosed herein, may be equal to or less than 2.5 wt%, such as equal to or less than 1.0, 0.5, 0.1, 0.05, 0.01, or 0.005 wt%. As the thickener, in contrast to water, will not evaporate, a low content of thickener is preferred. However, the content has to be high enough to noticeably affect the viscosity of the composition and give the desired effects described above. Accordingly, the lubricious aqueous composition may comprise at least 0.005 wt% of the thickener, such as at least 0.01 or 0.1 wt%.

A thickener, which is water-soluble or which swells in the presence of water to form a gel, will be easy to wash or wipe off skin without leaving any oily residue. Furthermore, it will not give rise to oily spots or stains on clothes, sheets or towels worn or used during, or after, massage.

The presence of least one compound forming a shear-thickening fluid when mixed with water will provide the aqueous composition with lubricous properties, thus less thickener, which also has may have lubricous properties, may be used. Without being bound to any theory, it is believed that the lubricous properties of the shear-thickening fluid are due to the formation of two substantially non-moving thin layers of the aqueous composition at surfaces gliding over each other. The presumed presence of such substantially non-moving layers may be explained by the non-Newtonian properties, i.e. shear-thickening properties, provided by the compound forming a shear-thickening fluid when mixed with water. Such layers effectively protect sensitive skin areas in contact with each over or in contact with a sex toy, and at the same time provide means for very close interaction.

According to one embodiment, a compound forming a shear-thickening fluid when mixed with water is a water soluble polymer. Further, such a polymer does typically have a high molecular weight, such as a molecular weight of at least 100,000 Dalton (Da), such as at least 500,000, at least 1,000,000 Da or even at least 5,000,000 Da. In addition, such a polymer is typically a linear, i.e. non-branched, polymer.

One preferred example of polymers having such properties is non-ionic, high molecular weight, water-soluble poly(ethylene oxide) polymers (PEO-polymers) having a molecular weight of at least 100,000 Da. Such polymers are sold by the Dow Chemical Company under the trademark PolyOx. A preferred type of PolyOx is PolyOx WSR 301.

As stated above, it is preferred that the compound forming a shear-thickening fluid when mixed with water, is pharmaceutically acceptable. In relation to this, it is preferred that the compound forming a shear-thickening fluid when mixed with water has such a high molecular weight that it can not penetrate human skin. Any possible side effects are prevented if the compound, forming a shear-thickening fluid when mixed with water, never enters the human body.

Similar to cross-linked polyacrylate, high molecular weight, water-soluble poly(ethylene oxide) polymers are in principle non-degradable by bacteria. Thus, they will not promote growth of bacteria in the vagina or disturb the natural bacterial flora. By using low amounts of non-biodegradable compound forming a shear-thickening fluid when mixed with water, such as water-soluble poly(ethylene oxide) polymers, the need to add preservatives may be dispensed with. Further, the composition may not promote bacterial growth to any extent.

The amount of the compound, forming a shear-thickening fluid when mixed with water, in the lubricious aqueous composition as disclosed herein may be equal to or less than 2.5 wt%, such as equal to or less than 1.0, 0.5, 0.1, 0.05, 0.01, or 0.005 wt%. As the compound forming a shear-thickening fluid when mixed with water, in contrast to water, will not evaporate, a low content of the compound forming a shear-thickening fluid when mixed with water is preferred, so that a minimum of compound is left if/when the water of the composition has evaporated. However, the content has to be high enough to noticeably affect the viscosity of the composition. Accordingly, the lubricious aqueous composition may comprise at least 0.005 wt% of the compound, forming a shear-thickening fluid when mixed with water, such as at least 0.01 or 0.1 wt%.

The presence of the two different compounds which give rise to fluids with different non-Newtonian properties (i.e. shear-thickening vs. shear-thinning) when mixed with water, will provide the lubricious aqueous composition with properties making it useful as a lubricant for sexual intercourse, masturbation and massage, as denoted above. However, it should be understood that the thickener does not have to be non-Newtonian.

In addition, the lubricious aqueous composition may, according to an embodiment, comprise a preservative. The preservative may be selected from pharmaceutically acceptable preservatives. Although, the pH of the lubricious aqueous composition typically is adjusted to be slightly acidic, such as between 4 and 5, it may still be preferred to add a preservative in order to provide the composition with prolonged shelf-life.

The amount of the preservative should be high enough to provide a preservative effect. As the efficacy of preservatives varies, the amount of a specific preservative effect needed to get a preservative effect varies, as is readily understood by the one skilled in the art. The amount of the preservative may be at least 0.1 wt%, such as at least 0.2, 0.5 or 1.0 wt%.

One example of a preferred preservative is a mixture of sodium benzoate and potassium sorbate. The amount of the preservative should be high enough to provide a preservative effect. If using such a combination, it is preferred if at least 0.01 wt%, such as at least 0.1 wt%, of each of the species are used. A mixture of sodium benzoate and potassium sorbate is available as an aqueous composition under the trademark Euxyl® K 712. Euxyl® K 712 is an aqueous solution comprising about 15wt% potassium sorbate and about 30 wt% sodium benzoate.

As a too high amount of preservative may give rise to side effects or even, however very unlikely, affect the rheological properties of the lubricious aqueous composition, it is preferred that the composition comprises less than 5 wt%, such as less than 2.5 wt%, less than 1.0 wt% or even less than 0.5 wt%, of the preservative. Further, the amount of preservative may also be affected by regulatory legislation.

According to an embodiment, the lubricious aqueous composition essentially consists of essentially non-biodegradable components and water, thus not promoting growth of microorganisms.

As denoted above, the need to add preservatives may be dispensed with if the lubricious aqueous composition only comprises components being essential non-biodegradable. As water may contain small amount of organic compounds, which may promote growth of microorganisms, the water used to produce the lubricious aqueous composition may be purified water. Examples of purified water includes distilled water, water purified by reverse osmosis. According to an embodiment, the water used to obtain the lubricious aqueous composition has a total organic carbon (TOC) content of 20 µg/L or less, such as 5 µg/L or less.

The Total Organic Carbon (TOC) is commonly defined as any compound containing a carbon atom except for CO₂, and related substances such as carbonate, bicarbonate and the like. As carbonates are considered to be fully oxidized, they do not form part of the constituents of TOC. Considering this exception to the definition of TOC, an alternative definition of TOC might be total oxidizable carbon.

According to embodiment, TOC, as used herein, is to be determined in accordance with the method 415.3 disclosed in *"DETERMINATION OF TOTAL ORGANIC CARBON AND SPECIFIC UV ABSORBANCE AT 254 nm IN SOURCE WATER AND DRINKING WATER"* (EPA Document #: EPA/600/R-05/055) issued by National Exposure Research Laboratory Office of Research and Development U.S. Environmental Protection Agency (EPA) Cincinnati, Ohio.

The pH of the lubricious aqueous composition should preferably be slightly acidic, i.e. below 7.0. An acidic pH will enhance the shelf-life of the product. Furthermore, the rheological properties, especially if a crosslinked polyacrylate is used as thickener, may depend on the pH of the lubricous aqueous composition. An aqueous solution comprising cross-linked polyacrylates may further be opalescent at a low pH. Accordingly, it is preferred if the pH of the composition is between 3 and 5, such as between 3 and 4 or between 4 and 5, eg. between 4.7 and 4.9. A transparent composition is typically desirable since it allows good visibility during massage and leaves a minimum of visible traces.

As indicated above the pH may affect the rheological properties of a lubricant comprising crosslinked polyacrylate as thickener. An increase in pH will increase the viscosity of the lubricant.

While the pH of in the vagina of a healthy woman is 3.8 to 4.5, the pH of semen is typically 7.2 to 8.0. Thus, the pH in the vagina will increase as semen is ejaculated into the vagina. The increase in pH, will accompanied by an increase in the viscosity of lubricant. Hence, the sperm motility will be obstructed.

In order to obtain a pH of 3 to 5, it may be necessary to adjust the pH of the lubricious aqueous composition by adding an acid or a base. Preferably, sodium hydroxide is used to adjust the pH. Sodium is a suitable counter-ion and hydroxide will give water when neutralized.

If the lubricant is to be used as a sex lubricant, a pH close the natural pH of the vagina is preferred. The normal vaginal pH is 3.8 to 4.5. Thus, the pH of the lubricious aqueous composition may, according to an embodiment, be 3.5 to 4.5.

Such a lubricious aqueous composition as disclosed herein may also be useful as a lubricant to be used for massage.

Further, it is envisaged that such a lubricious aqueous composition, as disclosed herein, may be useful as sex lubricant having contraceptive properties. In addition to the pH related effect disclosed herein above, a composition having shear thickening properties, such as the lubricious aqueous composition as disclosed herein, is believed to efficiently prevent sperms from moving, as any movement will give rise to an increased viscosity of composition in the vicinity of the sperm.

Also, the overall lower viscosity of the composition (as compared to said increased viscosity) makes the composition relatively easily distributable in the vagina to prevent sperms from movement anywhere in the vagina. Thus, a sperm is prevented from swimming from the vagina to the ovary after being ejaculated, if a lubricious aqueous composition comprising a compound forming shear-thickening fluid when mixed with water is present within the vagina. Hence, conception may be avoided. Accordingly, such a lubricant as disclosed herein may be used to obstruct sperm motility. In addition of being a useful property of a sex lubricant, the sperm motility obstructing property may also be useful when studying live sperms in microscope.

Such a sex lubricant for sexual activities as disclosed herein may be used alone. However, it may preferably be combined with other contraceptives, such as condoms or pessaries.

One preferred embodiment relates to a lubricious aqueous composition comprising:
- at least 96 wt%, such at least 98, 99 or 99.5 wt% water;
- between 0.01 and 2 wt%, such as between 0.05 and 1.0 wt%, of crosslinked polyacrylates;
- between 0.01 and 2 wt%, such as between 0.05 and 1.0 wt%, of a high molecular weight, water-soluble poly(ethylene oxide) polymer having molecular weight of at least 100,000 Da, such as at least 500,000 1,000,000 or 5,000,000 Da;
- pharmaceutical acceptable preservatives providing a preservative effect, wherein said preservatives includes potassium sorbate and sodium benzoate; and
- sodium hydroxide;
the composition having a pH of between 4 and 5, such as between 4.7 and 4.9.

Another preferred embodiment relates to a lubricious aqueous composition, for use a sex lubricant, essentially consisting of 98 to 99.9 wt% water, 1 to 0.05 wt% of an essentially none bio-degradable thickener, such as a high weight polymer of crosslinked polyacrylate, eg. Carbopol® Ultrez 21, and 1 to 0.05 wt% of essentially none bio-degradable compound forming a shear-thickening fluid when mixed with water, such as high molecular weight, water-soluble poly(ethylene oxide) polymers having a molecular weight of at least 100,000 Da, eg. PolyOx WSR 301. Such a composition may have a pH of 3.8 to 4.5. Further, the water used to obtain the composition may have a total organic carbon content (TOC) of 20 µg/L or less, such as 5 µg/L or less. As such composition comprises only essentially non-biodegradable components, it will not promote growth of micro-organisms. In addition, the absence of any preservative will minimize the affect on the natural urogenital flora of the woman. Further, as its pH corresponds to the normal pH of the human vagina and as it does not comprise any preservatives, it will not interfere with or affect the natural urogenital flora of the woman. Thus, such a lubricious aqueous composition may advantageously be used as sex lubricant.

Although the present invention has been described above with reference to (a) specific embodiment(s), it is not intended to be limited to the specific form set forth herein. Rather, the invention is limited only by the appended claims and, other embodiments than the specific above are equally possible within the scope of these appended claims.

In the claims, the term "comprises/comprising" does not exclude the presence of other elements or steps. Additionally, although individual features may be included in different claims and/or embodiments, these may possibly advantageously be combined, and the inclusion in different claims and/or embodiments does not imply that a combination of features is not feasible and/or advantageous. In addition, singular references do not exclude a plurality.

The terms "a", "an", "first", "second" etc. do not preclude a plurality. Reference signs in the claims are provided merely as a clarifying example and shall not be construed as limiting the scope of the claims in any way.

### Experimental

Although the present invention below is further described with reference to specific examples, it is not intended to be limited to the specific form set forth therein. Rather, the invention is limited only by the accompanying claims.

### Example 1 - Dissolvation in water of nonionic, high molecular weight water-soluble poly(ethylene oxide) polymers (PolyOx™)

PolyOx WSR 301 powder (6.5 kg; The Dow Chemical Company) was sifted and filled into the powder vessel. Using a vibration feeder, the PolyOx was fed into a water screen, obtained by feeding water to a dispenser. The mixture comprising PolyOx WSR 301 dissolved in 1000 L de-ionized water was stirred to obtain a solution suitable to be used for production of a shear-thickening solution.

### Example 2 - Preparation of lubricious aqueous composition

About 235 L de-ionized water was filled into a water vessel and 3 kg of Carbopol Ultrez 21 powder (Lubrizol Advanced Materials Europe BVBA) was spread over the water surface. After about 30 minutes, when the powder had been wet, the mixture of water and Carbopol was carefully stirred for about 2 minutes avoiding formation of foam. Then, 250 L of the shear-thickening solution from example 1 was carefully added and the mixture was stirred to form a thickened polymer solution. Subsequently, 4.39 kg Euxyl® K 712 (Schylke & Mayr), an aqueous solution comprising sodium benzoate and potassium sorbate, was added to the stirred thickened polymer solution. Finally, 0.46 kg NaOH dissolved in 10 L water was added to the stirred thickened polymer solution to adjust its pH-value. The pH-adjusted solution was then stirred for about 60 minutes until the liquid/gel was clear. The pH of the solution, which should be about 4.7 to 4.9, was then verified by measurement where after the liquid/gel was filtered through a straining cloth to obtain a lubricious aqueous composition free from lumps and ready for use as a lubricant for massage.

### Example 3 - long term storage.

A lubricious aqueous composition comprising PolyOx WSR 301 and Ultrez 21, but no preservative, was prepared. The pH of the composition was adjusted to about 4. The composition was packed into plastic tubes. After storage for more than a year, the content of microorganisms in the composition was analyzed. The composition was found to comprise a low amount of living bacteria. However, despite the absence of preservative, it could be concluded the number of colony forming units had not increased during the storage, thus confirming the non-biodegradability of the gel.

## Claims

1. A lubricious aqueous composition, comprising at least one thickener and at least one compound forming a shear-thickening fluid when mixed with water, which solution comprises at least 95 wt% water, wherein said thickener is a water insoluble hydrophilic compound which swells in the presence of water to form a gel, and said compound forming a shear-thickening fluid when mixed with water is a non-ionic, high molecular weight, water-soluble poly(ethylene oxide) polymer having molecular weight of at least 100,000 Da.

2. The composition according to claim 1, wherein the composition comprises at least 98 wt% water.

3. The composition according to any one of the preceding claims, wherein said thickener is a compound forming a shear-thinning fluid when mixed with water.

4. The composition according to any one of the preceding claims, wherein said thickener is cross-linked polyacrylate.

5. The composition according to any one of the preceding claims, wherein the lubricious aqueous composition comprises between 0.01 and 1 wt% of said thickener.

6. The composition according to any one of the preceding claims, wherein the lubricious aqueous composition comprises between 0.01 and 1 wt% of said compound forming a shear-thickening fluid when mixed with water.

7. The composition according to any one of the preceding claims, wherein the composition further comprises pharmaceutical acceptable preservative.

8. The composition according to any one of the preceding claims, wherein the water used to obtain said lubricious aqueous composition has a total organic carbon content of 20 µg/L or less.

9. The composition according to any one of the preceding claims, wherein the pH of the composition is between 3 and 5.

10. The composition according to any one of the preceding claims, wherein said lubricious aqueous composition essentially consists of 98 to 99.9 wt% water, 1 to 0.05 wt% of said thickener and 1 to 0.05 wt% of said compound forming a shear-thickening fluid when mixed with water.

11. The composition according to any one of the preceding claims, wherein said lubricious aqueous composition essentially consists of essentially non-biodegradable components and water.

12. Use of a composition according to any one of the preceding claims as a sex lubricant.

13. Use of a composition according to any one of the claims 1 to 11 for the manufacture of a sex lubricant.

14. Use of a composition according to any one of the claims 1 to 11 to obstruct sperm motility.

15. Use of a composition according to any one of the claims 1 to 11 for the manufacture of a sperm motility obstructing composition.

## Patentansprüche

1. Gleitfähige wässrige Zusammensetzung, umfassend mindestens ein Verdickungsmittel und mindestens eine Verbindung, die ein scherverdickendes Fluid bildet, wenn es mit Wasser vermischt wird, in welche die Lösung mindestens 95 Gew.% Wasser umfasst, wobei das Verdickungsmittel eine wasserunlösliche hydrophile Verbindung ist, die in Gegenwart von Wasser schwellt, um ein Gel zu bilden, und die Verbindung, die ein scherverdickendes Fluid bildet, wenn es mit Wasser vermischt wird, ein nicht ionisches, hochmolekulargewichtiges, wasserlösliches Poly(ethylenoxid) Polymer ist, die ein Molekulargewicht von mindestens 100.000 Da aufweist.

2. Zusammensetzung nach Anspruch 1, wobei die Zusammensetzung mindestens 98 Gew.% Wasser umfasst.

3. Zusammensetzung nach irgendeinem der vorhergehenden Ansprüche, wobei das Verdickungsmittel eine Verbindung ist, die ein scherverdünnendes Fluid bildet, wenn es mit Wasser vermischt wird.

4. Zusammensetzung nach irgendeinem der vorhergehenden Ansprüche, wobei das Verdickungsmittel vernetztes Polyacrylat ist.

5. Zusammensetzung nach irgendeinem der vorhergehenden Ansprüche, wobei die gleitfähige wäßrige Zusammensetzung zwischen 0,01 und 1 Gew.% des Verdickungsmittels umfasst.

6. Zusammensetzung nach irgendeinem der vorhergehenden Ansprüche, wobei die gleitfähige wäßrige Zusammensetzung zwischen 0,01 und 1 Gew.% der Verbindung umfasst, die ein scherverdickendes Fluid bildet, wenn es mit Wasser vermischt wird.

7. Zusammensetzung nach irgendeinem der vorhergehenden Ansprüche, wobei die Zusammensetzung ferner ein pharmazeutisches annehmbares Konservierungsmittel umfasst.

8. Zusammensetzung nach irgendeinem der vorhergehenden Ansprüche, wobei das für den Erhalt der gleitfähigen wäßrigen Zusammensetzung verwendete Wasser einen gesamten organischen Kohlenstoffgehalt von 20 µg/ L oder weniger aufweist.

9. Zusammensetzung nach irgendeinem der vorhergehenden Ansprüche, wobei der pH-Wert der Zusammensetzung zwischen 3 und 5 ist.

10. Zusammensetzung nach irgendeinem der vorhergehenden Ansprüche, wobei die gleitfähige wässrige Zusammensetzung im Wesentlichen aus 98 bis 99,9 Gew.% Wasser, 1 bis 0,05 Gew.% des Verdickungsmittels und 1 bis 0,05 Gew.% der Verbindung besteht, die ein scherverdickendes Fluid bildet, wenn sie mit Wasser vermischt wird.

11. Zusammensetzung nach irgendeinem der vorhergehenden Ansprüche, wobei die gleitfähige wässrige Zusammensetzung im Wesentlichen aus nicht abbaubaren Komponenten und Wasser besteht.

12. Verwendung einer Zusammensetzung nach irgendeinem der vorhergehenden Ansprüche als ein Sexschmiermittel.

13. Verwendung einer Zusammensetzung nach irgendeinem der Ansprüche 1 bis 11 für die Herstellung von einem Sexschmiermittel.

14. Verwendung einer Zusammensetzung nach irgendeinem der Ansprüche 1 bis 11, um Spermamotilität zu behindern.

15. Verwendung einer Zusammensetzung nach irgendeinem der Ansprüche 1 bis 11 für die Herstellung einer Spermamotilität-behindernden Zusammensetzung.

## Revendications

1. Composition aqueuse lubrifiante, comprenant au moins un agent épaississant et au moins un composé formant un fluide se viscosifiant par cisaillement lorsqu'il est mélangé à de l'eau, laquelle solution comprend au moins 95 % en poids d'eau, dans laquelle ledit agent épaississant est un composé hydrophile insoluble dans l'eau qui gonfle en présence d'eau pour former un gel, et ledit composé formant un fluide se viscosifiant par cisaillement lorsqu'il est mélangé à de l'eau est un polymère poly(oxyde d'éthylène) soluble dans l'eau, de haut poids moléculaire, non ionique, ayant un poids moléculaire d'au moins 100 000 Da.

2. Composition selon la revendication 1, dans laquelle la composition comprend au moins 98 % en poids d'eau.

3. Composition selon l'une quelconque des revendications précédentes, dans laquelle ledit agent épaississant est un composé formant un fluide rhéofluidifiant lorsqu'il est mélangé à de l'eau.

4. Composition selon l'une quelconque des revendications précédentes, dans laquelle ledit agent épaississant est un polyacrylate réticulé.

5. Composition selon l'une quelconque des revendications précédentes, dans laquelle la composition aqueuse lubrifiante comprend entre 0,01 et 1 % en poids dudit agent épaississant.

6. Composition selon l'une quelconque des revendications précédentes, dans laquelle la composition aqueuse lubrifiante comprend entre 0,01 et 1 % en poids dudit composé formant un fluide se viscosifiant par cisaillement lorsqu'il est mélangé à de l'eau.

7. Composition selon l'une quelconque des revendications précédentes, dans laquelle la composition comprend en outre un conservateur pharmaceutique acceptable.

8. Composition selon l'une quelconque des revendications précédentes, dans laquelle l'eau utilisée pour obtenir ladite composition aqueuse lubrifiante a une teneur totale en carbone organique de 20 µg/l ou moins.

9. Composition selon l'une quelconque des revendications précédentes, dans laquelle le pH de la composition est compris entre 3 et 5.

10. Composition selon l'une quelconque des revendications précédentes, dans laquelle ladite composition aqueuse lubrifiante est constituée essentiellement de 98 à 99,9 % en poids d'eau, de 1 à 0,05 % en poids dudit agent épaississant et de 1 à 0,05 % en poids dudit composé formant un fluide se viscosifiant par cisaillement lorsqu'il est mélangé à de l'eau.

11. Composition selon l'une quelconque des revendications précédentes, dans laquelle ladite composition aqueuse lubrifiante est constituée essentiellement de composants sensiblement non biodégradables et d'eau.

12. Utilisation d'une composition selon l'une quelconque des revendications précédentes comme lubrifiant pour rapports sexuels.

13. Utilisation d'une composition selon l'une quelconque des revendications 1 à 11 pour la fabrication d'un lubrifiant pour rapports sexuels.

14. Utilisation d'une composition selon l'une quelconque des revendications 1 à 11 pour entraver la motilité des spermatozoïdes.

15. Utilisation d'une composition selon l'une quelconque des revendications 1 à 11 pour la fabrication d'une composition entravant la motilité des spermatozoïdes.
